# EUROPEAN PATENT APPLICATION

(11) **EP 0 574 621 A1**
(43) Date of publication of application: **22.12.1993**
(21) Application number: 92305046.2
(22) Date of filing: 02.06.1992
(51) Int. Cl.: G01N 33/86, C07K 15/00

(54) **Bioassay for Von Willebrand's disease**

(71) Applicant: FUJITA HEALTH UNIVERSITY, Toyoake, Aichi 470-11 (JP); GENZYME CORPORATION, Cambridge, Massachusetts 02139-1562 (US)
(72) Inventor: Titani, Koiti, Kasugai-City, Aichi 487 (JP); Fujimura, Yoshihiro, Nara-city 631 (JP)
(74) Representative: Froud, Clive

(57) **Abstract**

Inter alia a method for detecting the presence of von Willebrand factor in a serum or plasma sample characterised in that it comprises:
(a) providing purified botrocetin;
(b) contacting the botrocetin with the serum or plasma;
   and
(c) detecting the reaction of the botrocetin with the serum or plasma as an indication of the amount of von Willebrand factor in the serum or plasma, is disclosed, as is a kit for such bioassay.

## Description

This invention relates to an improved method for diagnosing von Willebrand's disease using a purified form of botrocetin, a platelet aggregation factor found in the venom of the snake Bothrops jararaca.

Vascular damage, due to an event such as surgery, may result in the exposure of the subendothelium layer to circulating blood. Blood contains the plasma glycoprotein von Willebrand factor (vWF) which mediates platelet adhesion to the exposed subendothelium, especially at the relatively higher shear rates prevailing in arteries and the microcirculation system. Although the exact mechanism for platelet adhesion mediation is not understood, it is believed that vWF from the plasma binds to injured blood vessel walls, after which it interacts with a specific receptor termed the glycoprotein Ib - glycoprotein IX complex embedded in the platelet membrane. Patients having a genetic alteration of vWF are diagnosed as having von Willebrand's disease (vWD). vWD has been categorized into three distinct types: Type I, characterized by reduced plasma levels of vWF; Type II, characterized by a disfunctional vWF whereby the plasma concentration of vWF is essentially normal but the structure of the vWF has a reduced number of multimers; and Type III, characterized by the substantial absence of vWF molecules.

The antibiotic ristocetin has been widely used as a diagnostic aid to assay for the presence of vWF in vitro. The assay methodology measures the rate of platelet aggregation using visual or optical means. The use of ristocetin as an active component of such assays is unsatisfactory in several respects, however. First, ristocetin activity has only been demonstrated using human platelets. Platelets from other species respond poorly in this assay. Second, ristocetin activity is dependent on the presence of vWF multimers, and plasma from patients suffering from vWD (Type II) are negative in a ristocetin-based assay. Therefore, ristocetin cannot distinguish between Type I, Type II or Type III vWD. Third, ristocetin, because of the high concentrations used in the in vitro assay, can in some instances cause precipitation of general plasma proteins, interfering with the detection of platelet aggregation.

Reed et al. discuss, in the Proceedings of the National Academy of Sciences, 75, 4514 (1978), a partially purified substance from the venom of the snake Bothrops jararaca which has the property of promoting platelet aggregation in the presence of vWF. Reed et al. suggest the use of this substance as a substitute for ristocetin, and named it "botrocetin". Even though several functional differences between these two compounds has been observed, both ristocetin and botrocetin are known to induce vWF binding to platelet glycoprotein Ib. Moreover, the binding of vWF to glycoprotein Ib in the presence of either ristocetin or botrocetin is mediated by the same domain of the vWF molecule. Unlike ristocetin, however, botrocetin can promote platelet aggregation in a variety of animal species other than humans, botrocetin can promote platelet aggregation in plasma from patients with Type II vWD, and botrocetin does not appear to cause non-specific plasma protein precipitation. Since the treatment of vWD is based on the type of disorder exhibited by the patient (Type I, II or III), it would be advantageous to have assay reagents which can be used to distinguish between these three types. A botrocetin-based test, together with the ristocetin test, could distinguish the particular type of vWD. To develop such a test far clinical use would require a well characterized and purified botrocetin reagent.

Several attempts have been made to obtain a purified form of botrocetin. Reed et al. in Blood, vol. 74, page 1031 (1989), reported that a purified botrocetin prepared by anion-exchange chromatography shows a single band at 26.5 kilodaltons (kDa) using polyacrylamide gel electrophoresis in the presence of sodium dodecyl sulfate, and demonstrated that it binds to purified vWF and farms an activated complex that, in turn, binds to glycoprotein Ib. Andrews et al. in Biochemistry, vol. 28, page 8317 (1989), described a purified botrocetin product having a 25 kDA disulfide-linked dimer with apparent subunits of 14 kDA and 14.5 kDA. N-terminal sequencing of this dimer yields a single protein sequence. A tryptic fragment of vWF bound to beads coated with this product, whereas glycoprotein Ib fragments containing the vWF binding site did not, indicating that botrocetin mediated platelet aggregation by alteration of the vWF.

It is therefore an aspect of this invention to provide a botrocetin compound which is of sufficiently high purity to be useful in a bioassay for vWF present in the serum or plasma of a patient.

According to the present invention, an improved bioassay for von Willebrand factor (vWF) utilizes a highly purified form of botrocetin. The botrocetin is obtained by purification of a crude extract of venom from the snake Bothrops jararaca. Purification can be advantageously achieved utilizing anion-exchange chromatography, with a final separation accomplished by hydrophobic interaction. The purified material can be further fractionated to yield a one-chain protein and a two-chain protein. Although both of these proteins are generically described herein as "botrocetin", the two-chain protein has a platelet aggregation-promoting activity of at least an order of magnitude greater than the corresponding one-chain protein. The reason for this enhanced activity is unknown. The fractionation procedure utilizes reverse phase chromatography and different solubilities in hydrophobic solvents. Alternatively, the two-chain protein can be prepared by fractionating the crude venom from the snake Bothrops jararaca by anion-exchange chromatography, and the resulting material can be adsorbed to an immobilized glycoprotein and eluded. The two-chain compound has an alpha subunit and a beta subunit with amino acid sequences as shown in SEQ ID NO:1 and SEQ ID NO:2.

The purified botrocetin is useful as a component of an assay kit for the bioassay of von Willebrand factor. Such an assay kit also includes suitable buffering agents and instructions for their use. An assay kit specific for von Willebrand factor, Type II, additionally includes purified ristocetin as an active component.

The assay procedure involves contacting the purified botrocetin with a sample of serum or plasma from a patient, and observing the rate of platelet aggregation in the serum or plasma due to the reaction of the botrocetin with the von Willebrand factor present in the sample. The rate of platelet aggregation may be recorded visually or optically by means of suitable instrumentation. In this manner, the presence of von Willebrand factor, Types I or III, in a sample can be readily determined.

An assay for von Willebrand factor, Type II, follows the above procedure, and additionally includes the reaction of purified ristocetin with a second portion of the serum or plasma. A comparison of the rate of platelet aggregation in the presence of botrocetin (and ristocetin) is then made.

### Referring to the accompanying illustrations:

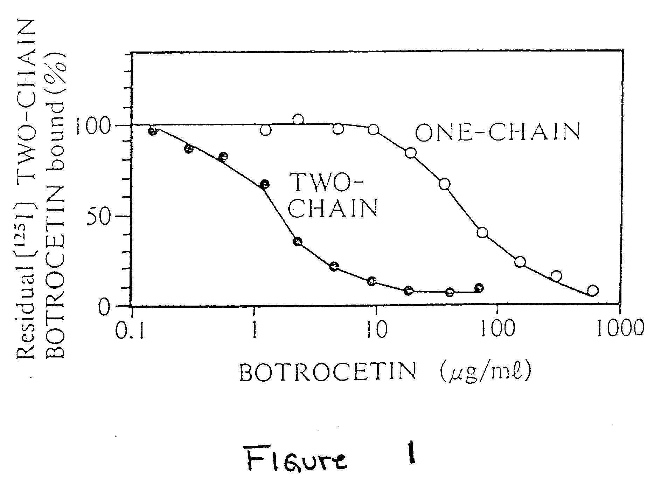

Figure 1 is a diagrammatic representation of the competitive binding of one-chain botrocetin and two-chain botrocetin to von Willebrand factor.

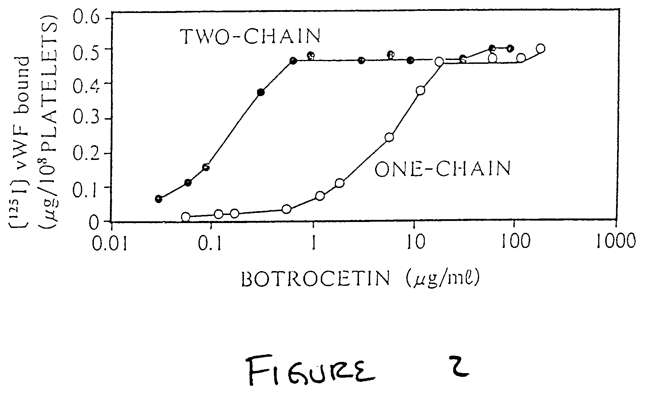

Figure 2 is a diagrammatic comparison of one-chain and two-chain botrocetin activity ¹²⁵I - von Willebrand factor binding to platelet glycoprotein Ib.

Botrocetin, which is purified as described herein, is a primary component of a bioassay for von Willebrand disease. The botrocetin is obtained from the venom of the snake Bothrops jararaca by purification of the crude venom extract. Purification can be accomplished by fractionation using anion-exchange chromatography, such as diethyl amino chromatography, followed by size exclusion chromatography, and a final purification by hydrophobic high-performance liquid chromatography. The product obtained from this purification scheme is a mixture of both one-chain and two-chain botrocetin. The one-chain protein can be separated from the two-chain protein by elution using reverse-phase high-performance liquid chromatography. Alternatively, the two-chain botrocetin can be prepared by fractionating the crude snake venom using anion-exchange chromatography, adsorbing the botrocetin on an immobilized glycoprotein, and specifically eluting the two-chain botrocetin. Both of these preparatory methods yield a two-chain molecule having a molecular mass of 27 Kda with the amino acid sequence shown in SEQ ID NO: 1 and SEQ ID NO:2.

The purified botrocetin which is useful in this invention can also be prepared by recombinant DNA technology. It is particularly advantageous to prepare the two chain protein in this manner since the two-chain protein has approximately 30 times the activity of the one-chain protein in promoting platelet aggregation. Consequently, the availability of the two-chain protein in significant quantities enables the preparation of a bioassay for von Willebrand disease having improved sensitivity and accuracy. Recombinant techniques may be used to manufacture the two-chain protein due to the identification of the complete amino acid sequence. With the amino acid sequence in hand, one skilled in this art can readily isolate DNA sequences encoding this protein using known molecular biology procedures. This DNA could then be engineered into bacterial, yeast or mammalian expression vectors to produce large quantities of the protein.

For example, DNA oligonucleotides may be synthesized based on this protein sequence, particularly in the region of amino acid number 12 to amino acid number 23 of the alpha subunit. This region contains amino acids which have comparatively few codon redundancies. The amino acid region 16 to 25 of the beta subunit can also be advantageously used to design oligonucleotides for the some reason.

These oligonucleotides can be radiolabeled and used as probes to screen a Bothrops jararaca genomic or complementary DNA library which is constructed using standard techniques. See, for instance, J. Sambrook et al., Molecular Cloning: A Laboratory Manual, Cold Spring Harbor Press (1989). In the event that each subunit of the two-chain protein is encoded by a separate gene, oligonucleotide probes for bath the alpha and beta subunits may be used to screen genomic or complementary libraries independently.

Once genomic or complementary DNA sequences have been isolated which encode the alpha and beta subunits, these sequences can be engineered into DNA vectors far expression in bacterial, yeast and animal cell systems. A wide variety of expression systems are available in the literature for possible use as expression vehicles for such vectors.

The production of increased amounts of botrocetin using recombinant technology has important implications for diagnostic test kits since increased amounts of starting material allow far higher purification yields and a more consistent final product.

The diagnostic procedure of this invention includes providing a purified botrocetin as described above, and contacting the botrocetin with the serum or plasma of a patient. Reaction of the botrocetin in the presence of von Willebrand factor in the serum or plasma results in platelet aggregation. The rate of platelet aggregation is an indication of the presence and relative amount of von Willebrand factor in the serum or plasma, and is therefore an indicator of von Willebrand's disease. The rate of platelet aggregation can be detected visually or using a suitable optical device. An assay specific for von Willebrand factor, Type II, includes the steps of contacting one portion of the serum or plasma with botrocetin, and a second portion of the plasma or serum with purified ristocetin, and comparing the rate of platelet aggregation in both instances.

The purified botrocetin of the present invention can be formulated using known procedures to prepare compositions suitable for storage and stability, facilitating the use and handling of the material.

**The following examples are intended to illustrate further various aspects of the present invention.**

The examples employ various materials and equipment obtained from bath commercial and private sources. A description of such materials and equipment is provided immediately below under the heading "General Methods".

### General Methods

Crude Bothrops jararaca venom (lots 75F03409, 68F0557, 98F0261, and 119-F0599), bovine fibrinogen (type I), and DFP (diisopropl fluorophosphate) were obtained from Sigma. TPCK (N-α-tosyl-phenylalanine chloromethyl ketone) -trypsin and α-chymotrypsin were purchased from Worthington. Thermolysin and pepsin were obtained from Seikagaku Kogyo and arginylendopeptidase was obtained from Takara Shuzo. Achromobacter protease I was a gift from Dr. T. Masaki at Ibaraki University, Ibaraki, Japan. Staphylococcus aureus V8 protease was obtained from Miles. BSA (bovine serum albumin), neuaminidase and endo F (endo-β-N-acetylglucosaminidase F) were obtained from Calbiochem. Cyanogen bromide and lodogen were obtained from Pierce. Carrier-free Na¹²⁵I was obtained from Amersham. DEAE (diethly aminoethyl) -Sepharose CL-6B gel and Phenyl-Superose HR5/5 column were products of Pharmacia-LKB. TSK G3000Sw and G2000Sw columns were obtained from Toyo Soda. Protein concentration was determined by dye-binding assay kit from Bio-Rad using BSA as a standard. Highly purified vWF was isolated from cryoprecipitate (gift from Nara Red Cross Blood Center, Nara Japan) as described by Fujimura et al, Journal of Biological Chemistry 261,381 (1986).

Protein labeling with ¹²⁵I was performed by the lodogen method described by Fraker and Speak, Biochemistry and Biophysics Research Communication 80, 849 (1978).

### Example 1 -Purification of one-chain botrocetin

Botrocetin was purified by anion-exchange chromatography followed by size exclusion and hydrophobic HPLC (high-performance liquid chromatography). All of the procedures in the anion-exchange step were performed at 4°C. Five hundred milligrams of the crude venom (lots 75F03409 and 68F0557) was dissolved in 50 ml of Buffer A (84 mM imidazole buffer containing 0.02% NaN₃ and 2 mM benzamidine, pH 7.4). The insoluble material was removed by centrifugation at 2,500 xg for 15 minutes. The supernatant was applied to a 2.6 x 35 cm column of DEAE-Sepharose CL-6B previously equillibrated with Buffer A. The column was washed with Buffer A for 18 hours at a flow rate of 50 ml/hour. The column was then washed with Buffer A containing 0.1 M NaCl for 12.5 hours. The column was then developed with a linear gradient from 0.1-0.7M NaCl in Buffer A, using 150 ml each of the initial and final buffer. Fractions (9ml) were collected and monitored for protein, fibrinogen clotting activity and the activity promoting ¹²⁵IvWF binding to platelets. The results of fractionation of crude Bothrops jararaca venom (500 mg) by anion-exchange chromatography on a DEAE-Sepharose CL-6B column indicate that more than 90% of the fibrinogen clotting activity present in the starting material eluted from the column at 0.1 M NaCl. By a gradient elution from 0.1 to 0.7M NaCl, three major peaks were obtained and pooled. One peak showed only residual fibrinogen clotting activity. The other two peaks showed activity for inducing vWF binding to platelets and vWF-dependent platelet aggregation, but no fibrinogen clotting activity. These two peaks were pooled, dialyzed against 0.1M NH₄HCO₃, pH 7.8 at 4°C for two days and lyophilized.

The lyophilized protein was dissolved in Buffer B (0.02M Tris-HCl and 0.5M NaCl, pH 6.8). Three milligrams of protein were separated by size exclusion HPLC on tandem columns of TSK G3000SW and G2000SW at a flow rate of 1.0 ml/min and collected in 1.0 ml fractions. A major protein peak was eluted which coincided with the activity promoting vWF binding to GP (glycoprotein) Ib. The fractions which retained the activity promoting vWF binding to platelets were pooled, dialyzed and lyophilized.

The lyophilized sample was dissolved in Buffer C (1.7 M (NH₄)₂SO₄), and then each 500 µg of protein was further purified by hydrophobic HPLC an a Phenyl-Superose HR 5/5 column previously equillibrated with Buffer C at a flow rate of 0.5 ml/min. After 5 minutes, a 1.7-0 M (NH₄)₂SO₄ gradient in Buffer B was started using 18 milliliters each of the initial and final buffer. Several protein peaks were eluted from the column and the activity promoting vWF binding corresponded to the major peak. The peak fractions were pooled, dialyzed and lyophilized and stored at -20°C until used.

### Example 2-Purification of two-chain botrocetin

Two chain botrocetin was purified according to the method described in Example 1 except that lots 98F0261 and 119F0599 of the crude venom were the source of the starting material. In addition, the two-chain botrocetin is separated from the one-chain botrocetin by reverse-phase HPLC on SynChropak RP-8 (C8). Essentially, the purified one-, two-chain botrocetin mixture is applied to the column and eluted with an acetonitrile gradient from 0 to 60%. The protein elution is monitored by absorption at 280 nm. Two peaks elute with the first being the one-chain botrocetin and the second being the two-chain botrocetin.

### Example 3-Purification of botrocetin using affinity chromatography

A second method for purification of two-chain botrocetin takes advantage of the newly discovered lectin-like activity of the protein. One-half gram of Bothrops jararaca venom is dissolved in 20 ml of 20 mM Tris-HCl, pH 7.5 and applied to a 50 ml DEAE-Sephacel column. The column is developed with a 0.1 - 1.0M NaCl gradient in 20 mM Tris, pH 7.5 and column fractions analyzed for vWF binding ond platelet aggregating activity. The active fractions were pooled and dialyzed into Buffer D (20 mM Tris-HCl, pH 7.5; 150 mM NaCl). CaCl₂ was then added to a final concentration of 2 mM.

A 10 ml Asialofetuin-Sepharose 4B column was prepared according to the method of Roff and Wang (Journal of Biological Chemistry 258, 10657 (1983)). The column was equillibrated with Buffer D containing 2 mM CaCl₂. The pooled active fractions were then applied to the column and the column washed with Buffer D containing 2 mM CaCl₂ until the absorption at 280 nm of the effluent was undetectable. The two-chain botrocetin was then eluted with Buffer D containing 10 mM EDTA. SDS-PAGE (polyacrylamide gel electrophoresis in the presence of sodium dodecyl sulfate) analysis of the EDTA-eluted fraction indicated that the band mobility was the same as the botrocetin purified in Example 2.

SDS-PAGE (4-20% gradient) analysis revealed that the purified one-chain botrocetin had a molecular mass of 28 Kda before and 32 Kda after reduction with DTT (dithiothreitol), indicating that this botrocetin is composed of a single polypeptide chain. In six different experiments, approximately 1-2.5 mg of highly purified one-chain botrocetin was obtained from 500 mg of the same lot of crude venom. The pl was estimated to be 4.0-4.1 by isoelectric focusing, and the extinction coefficient (E^{1%}, 1 cm) was 10.75. No proteolytic activity of purified botrocetin on vWF was observed when the incubation mixture was analyzed by SDS-Agarose gel electrophoresis.

Similar SDS-PAGE analysis of the two-chain botrocetin showed that it had a molecular mass of 27 Kda before and a 14.5/15 Kda doublet after reduction with DTT, indicating that this form of botrocetin is composed of two polypeptide chains linked by di-sulfide interactions.

### Example 4-Amino acid composition of one-chain and two-chain botrocetin

The elucidation of the amino acid compositions of one-chain and two-chain botrocetin preparations was desired in order to ascertain that they were two distinct proteins and not two forms of the same protein. Amino acid compositions were determined with a Hitachi Amino Acid Analyzer Model L-8500 or with a Waters Picotag system (Bidlingmeyer et al, Journal of Chromatography 336, 93(1984)) after 24 hour acid hydrolysis. The amino acid composition of the one-chain and two-chain botrocetin is shown in the following Table:

| Amino Acid Composition of Purified Botrocetin^{a} | | |
|---|---|---|
| Composition | One-Chain | Two-Chain |
| CAM-cys^{b} | 33.8 | 15.6 |
| Asx | 39.1 | 33.8 |
| Thr | 9.1 | 8.6 |
| Ser | 16.8 | 25.4 |
| Glx | 32.2 | 37.7 |
| Pro | 15.8 | 10.5 |
| Gly | 27.6 | 16.9 |
| Ala | 16.2 | 14.2 |
| Val | 12.2 | 19.8 |
| Met | 3.6 | 4.4 |
| Ile | 5.5 | 9.2 |
| Leu | 10.9 | 13.7 |
| Phe | 7.2 | 19.8 |
| Tyr | 14.8 | 13.4 |
| His | 8.3 | 5.1 |
| Lys | 19.5 | 22.4 |
| Arg | 4.9 | 7.5 |
| Trp | N.D.^{c} | N.D. |
| Total Residues | 278.0 | 278.0 |

| | | |
|---|---|---|
| ^{a}Values are expressed as residues/molecule, assuming a molecular mass of 32 kDa for both species. | | |
| ^{b}CAM-Cys was determined as CM-Cys by amino acid analysis. | | |
| ^{c}Not-determined. | | |

### Example 5-Amino terminal sequence of one-chain and two chain botrocetin.

The elucidation of the protein amino terminal sequence was desired to confirm that the one-chain and two-chain botrocetin molecules were two separate and distinct proteins. Amino acid sequences were determined with on Applied Biosystems Protein Sequencer Model 470A after blotting onto a polyvinylidene membrane (Motsudaira, Journal of Biological Chemistry 262,10035 (1987)) or after separation by reverse-phase HPLC. Cysteine and cystine content of the proteins were determined by first dissolving the proteins in buffer containing 0.3M Tris-HCl, pH 8.3 and 6M guanidine-HCl. Aliquots of the solution, either before or after reduction of disulfide bonds with tri-n-butylphosphin (Ruegg and Rudinger, Methods in Enzymology XLVII, 111(1977)), were treated with 4-vinylpyridine (Hermodson et al, Biochemistry 12, 3146 (1973)) and the content of S-pyridylethyl cysteine was quantitated by amino acid analysis. Neutral sugar content was estimated by the method of Dubois et al, Analytical Chemistry 28, 350 (1946).

The NH₂-terminus of the one-chain botrocetin was not blocked and the amino acid sequence of the first 25 residues was determined as shown in SEQ ID NO:3. In cycle 2, two residues, Ile and Val, were identified at almost equal quantities indicating the presence of a polymorphism at this position. The two-chain botrocetin was reduced and S-pyridylethylated and then separated into its component subunits by reverse-phase HPLC on a SynChropak RP-8 column. Although the NH₂-terminal sequences for the two subunits are similar, they are distinct, thus the two-chain botrocetin is a disulfide-linked heterodimer composed of two subunits having different sequences and distinct from that of the one-chain molecule.

### Example 6-Biological activity of one-chain and two-chain botrocetin

### A. Measurement of the botrocetin-vWF complex formation

Polystyrene microtiter plates with removable flat bottom wells (Immulon I Removawell strips, Dynatech Laboratories) were coated for 2 hours at room temperature with 100 µl of a solution of purified monoclonal antibody 2-2-9 IgG (10µg/ml in 0.05M bicarbonate buffer, pH 9.0). This monoclonal antibody binds to the carboxyl-terminal region of the vWF and has no effect on vWF binding to platelets in the presence of either ristocetin or botrocetin. The coating solution was removed and the plastic surface was covered with 200 mls of 1% BSA in TBS (0.05M Tris-buffered saline) for 1 hour at room temperature. The BSA solution was then removed and the wells washed 3 times with TBS containing 0.05% NP-40 (TBS-NP40). Formation of the botrocetin-vWF complex in fluid phase was obtained by mixing purified vWF (5 µg/ml), ¹²⁵I-two-chain botrocetin (3.2 µg/ml) and BSA (5 mg/ml) in an incubation volume of 120 µl in plastic Eppendorf tubes at room temperature. For competitive inhibition studies, various amounts of unlabeled one-chain or two-chain botrocetin were added to the incubation mixture. After 30 minutes, two 50 µl aliquots of each mixture were added into the 2-2-9 IgG coated wells in order to bind vWF through its carboxyl-terminal portion (Nishio et al, American Journal of Hematology 33, 261 (1990)). After an additional 30 minutes at room temperature, the wells were washed 5 times with 500 µl of TBS-NP40 and the radioactivity bound to the wells was measured to calculate the amount of radiolabeled botrocetin complexed to vWF. Non-specific binding was determined by omitting vWF from the incubation mixtures. Specific binding was calculated by subtracting non-specific from total binding.

¹²⁵I-labeled two-chain botrocetin and vWF formed a complex in solution, as shown by the observation that on anti-vWF monoclonal antibody could bind botrocetin only when vWF was present. The specific binding of ¹²⁵I-two-chain botrocetin to vWF was competitively inhibited in a dose-dependent manner by both the one-chain and two-chain botrocetin. The corresponding IC₅₀ values (namely the concentration unlabeled botrocetin necessary to inhibit binding of radiolabeled botrocetin to vWF by 50%) was 55 µg/ml for the one-chain and 1.7 µg/ml for the two-chain species. Thus, the two-chain botrocetin has a 32-fold higher affinity for vWF than the one-chain species.

### B. Botrocetin induced binding of vWF to platelet glycoprotein Ib.

Botrocetin induced vWF binding to plotelet glycoprotein Ib was measured by mixing formalin-fixed platelets (1 x 10⁸/ml) and ¹²⁵I-labeled vWF (final concentration 5 µg/ml) in an Eppendorf tube and the final volume adjusted to 112.5 µl with TBS. A 12.5 µl aliquot of each botrocetin sample, diluted 10-fold with TBS, was added to this mixture to initiate vWF binding to glycoprotein Ib. After 30 minutes incubation at room temperature, the mixture was divided into duplicate 50 µ aliquots and the radioactivity bound to the platelets was separated from free radioactivity by centrifugation for 5 minutes at 13.000 g through a 20% sucrose cushion using a Sarstedt microcentrifuge tube as previously described by Fujimura et al, Blood 70, 985 (1987). Non-specific binding was defined as the amount of radioactivity measured in the presence of a 50-fold excess of unlabeled vWF. Specific binding was calculated by subtracting the non-specific binding from the total.

The minimum concentration of the one-chain botrocetin necessary to induce vWF binding to glycoprotein Ib was 1 µg/ml, and the maximal binding was obtained with 17 µg/ml. Half maximal binding capacity (BM₅₀) was estimated to correspond to 5.5 µg/ml. In contrast, the BM₅₀ for the two-chain molecule was 0.16 µg/ml indicating a 34-fold greater activity than for the one-chain botrocetin. This is also 40-fold greater than the 6.5 µg/ml BM₅₀ reported by Andrews et al (Biochemistry 28, 8317 (1989)) for the two-chain botrocetin isolated by these researchers.

### C. Measurement of botrocetin induced platelet aggregation

Platelet aggregation was measured using a NKK aggregometer using 0.3 ml of platelet-rich plasma at 37°C as described by De Marco et al, Journal of Clinical Investigation 77, 1272 (1986).

Two final concentrations of purified one-chain botrocetin (10 and 50 µg/ml) were used in platelet aggregation studies. The platelet count was adjusted to 1 x 10⁸/ml for all samples, because this was the maximal count attainable in the two patients with Bernard-Soulier Syndrome who had a slight thrombocytopenia (0.7 - 1.4 x 10⁸/ml). The purified one-chain botrocetin induced aggregation in the platelet-rich plasma of a patient with Glanzmann Thrombasthenia. Thus, the effect of botrocetin is independent of the function of the glycoprotein IIb/IIIa complex, which is essential for platelet aggregation resulting from agonist-induced activation. In contrast, botrocetin had no effect on the platelet-rich plasma from the two patients with Bernard-Soulier Syndrome. These patients lack functional expression of the glycoprotein Ib-IX complex and, therefore, are defective in any activity dependent on vWF-glycoprotein Ib interaction.

## Claims

1. A method for detecting the presence of von Willebrand factor in a serum or plasma sample characterised in that it comprises:
(a) providing purified botrocetin;
(b) contacting the botrocetin with the serum or plasma;
and
(c) detecting the reaction of the botrocetin with the serum or plasma as an indication of the amount of von Willebrand factor in the serum or plasma.

2. A method as claimed in claim 1 wherein the von Willebrand factor detected is either Type I or Type III.

3. A method for detecting the presence of von Willebrand factor, Type II, in a serum or plasma sample characterised in that it comprises:
(a) providing purified botrocetin;
(b) contacting the botrocetin with a first portion of the serum or plasma;
(c) providing purified ristocetin;
(d) contacting the ristocetin with a second portion of the serum or plasma;
and
(e) detecting the reaction of the botrocetin and the ristocetin with the serum or plasma and comparing the reactions as an indication of the amount of von Willebrand factor, Type II, in the serum or plasma.

4. A method as claimed in any of claims 1 to 3 wherein the sample comprises human serum or plasma.

5. A method as claimed in any of claims 1 to 4 wherein the reaction of botrocetin with the serum or plasma results in platelet aggregation in the presence of von Willebrand factor and the rate of such platelet aggregation is recorded.

6. A method as claimed in any of claims 1 to 5 wherein the purified botrocetin is a two-chain molecule.

7. A kit for the bioassay of von Willebrand factor characterised in that it comprises purified botrocetin, optionally purified ristocetin, suitable buffering agents and instructions for use.


INFORMATION FOR SEQ ID NO:1:
(i) SEQUENCE CHARACTERISTICS:
(A) LENGTH: 133 Amino acids
(B) TYPE: Amino acid sequence
(C) STRANDEDNESS: Double
(D) TOPOLOGY: Linear

(ii) MOLECULE TYPE: Protein

(iii) SEQUENCE DESCRIPTION: Seq ID No:1:

ASP CYS PRO SER GLY TRP SER SER TYR GLU GLY ASN CYS TYR LYS
1               5                   10                  15

PHE PHE GLN GLN LYS Met ASN TRP ALA ASP ALA GLU ARG PHE CYS
                20                  25                  30

SER GLU GLN ALA LYS GLY GLY HIS LEU VAL SER ILE LYS ILE TYR
                35                  40                  45

SER LYS GLU LYS ASP PHE VAL GLY ASP LEU VAL THR LYS ASN ILE
                50                  55                  60

GLN SER SER ASP LEU TYR ALA TRP ILE GLY LEU ARG VAL GLU ASN
                65                  70                  75

LYS GLU LYS GLN CYS SER SER GLU TRP SER ASP GLY SER SER VAL
                80                  85                  90

SER TYR GLU ASN VAL VAL GLU ARG THR VAL LYS LYS CYS PHE ALA
                95                  100                 105

LEU GLU LYS ASP LEU GLY PHE VAL LEU TRP ILE ASN LEU TYR CYS
                110                 115                 120

ALA GLN LYS ASN PRO PHE VAL CYS LYS SER PRO PRO PRO
                125                 130

INFORMATION FOR SEQ ID NO:2:
(i) SEQUENCE CHARACTERISTICS:
(A) LENGTH: 125 Amino acids
(B) TYPE: Amino acid sequence
(C) STRANDEDNESS: Double
(D) TOPOLOGY: Linear

(ii) MOLECULE TYPE: Protein

(iii) SEQUENCE DESCRIPTION: Seq ID No:2:

ASP CYS PRO PRO ASP TRP SER SER TYR GLU GLY HIS CYS TYR ARG
1               5                   10                  15

PHE PHE LYS GLU TRP MET HIS TRP ASP ASP ALA GLU GLU PHE CYS
                20                  25                  30

THR GLU GLN GLN THR GLY ALA HIS LEU VAL SER PHEGLN SER LYS
35 40 45

GLU GLU ALA ASP PHE VAL ARG SER LEU THR SER GLU MET LEU LYS
50 55 60

GLY ASP VAL VAL TRP ILE GLY LEU SER ASP VAL TRP ASN LYS CYS
65 70 75

ARG PHE GLU TRP THR ASP GLY MET GLU PHE ASP TYR ASP ASP TYR
80 85 90

TYR LEU ILE ALA GLU TYR GLU CYS VAL ALA SER LYS PRO THR ASN
95 100 105

ASN LYS TRP TRP ILE ILE PRO CYS THR ARG PHE LYS ASN PHE VAL
110 115 120

CYS GLU PHE GLN ALA
125

INFORMATION FOR SEQ ID NO:3:
(i) SEQUENCE CHARACTERISTICS:
(A) LENGTH: 35 Amino acids
(B) TYPE: Amino acid sequence
(D) TOPOLOGY: Linear

(ii) MOLECULE TYPE: Protein

(iii) SEQUENCE DESCRIPTION: Seq ID No:3:

ILE ILE SER PRO PRO VAL CYS GLY ASN GLU LEU LEU GLU VAL GLY
1               5                   10                  15

GLU GLU CYS ASP CYS GLY THR PRO GLU ASN CYS GLN ASN GLU CYS
                20                  25                  30

CYS ASP ALA ALA THR
                35